# EUROPEAN PATENT SPECIFICATION

(11) **EP 1 267 984 B1**
(45) Date of publication and mention of the grant of the patent: **09.11.2005**
(21) Application number: 01918749.1
(22) Date of filing: 16.03.2001
(51) Int. Cl.: A61M 25/01

(54) **INTRODUCER SHEATH**
EINFÜHRUNGSHÜLSE
GAINE D'INTRODUCTION

(30) Priority: 21.03.2000 US 191058 P
(43) Date of publication of application: 02.01.2003
(73) Proprietor: Cook Incorporated, Bloomington, IN 47402-0489 (US); Sabin Corporation, Bloomington, Indiana 47402 (US)
(72) Inventor: DREWES, David, A., Jr., Bloomington, IN 47403 (US); EELLS, Scott, E., Bloomington, IN 47403 (US); GRAF, Matthew, M., Bloomington, IN 47403 (US)
(74) Representative: Lomas, Geoffrey Michael
(86) International application number: PCT/US2001/008432
(87) International publication number: WO 2001/070324

(56) References cited:
- WO-A-99/48548
- US-A- 4 657 024
- US-A- 5 045 072
- US-A- 5 300 048
- US-A- 5 948 489

## Description

The present invention relates generally to the field of medical devices and more particularly to introducer sheaths.

### Background of the Invention

An introducer sheath is utilized in the percutaneous placement of a guide wire or catheter into a blood vessel, and comprises a flexible tube that itself is introduced into the blood vessel over a dilator. Once in position, the dilator is removed from within the sheath and withdrawn from the patient, and the guide wire or catheter is inserted through the sheath into the patient. Such sheaths are of biocompatible polymeric material and preferably contain an amount of radiopaque material in the polymeric matrix, and include a short tapered distal tip portion. Sheaths should have sufficient radial rigidity to remain open or patent upon removal of the dilator, but be sufficiently flexible to permit manipulation without kinking, under conditions of normal use. Internal sheath diameters range from 4 French to 26 French (1.3 mm to 8.7 mm) to accommodate the outside diameters of dilators and catheters and wire guides to extend therethrough.

Introducer sheaths are known that include adjacent to the distal tip portion, a radiopaque marking distinct from the remainder of the sheath, to indicate through fluoroscopy the position of the distal tip portion of the sheath within the patient, to assure proper positioning. The sheath can be of fluorinated ethylene propylene (FEP) having about 5 to 40% by weight loading of barium filler. Introducer sheaths have been known that include an annular ring of radiopaque paint on the sheath adjacent to the distal tip. Also, such marking typically can be an annular band of platinum alloy, or tungsten or gold or the like that is secured within the outer surface of the sheath adjacent to the distal tip, as in the CHECK-FLO PERFORMER Introducer Sheath sold by Cook Incorporated, Bloomington, IN. The metal band is spaced approximately one-quarter inch from the distal tip and imparts substantial rigidity to the somewhat flexible sheath, where as it would be desirable for the sheath to flex sufficiently during positioning to temporarily assume an oval cross-section locally.

It has been known to provide catheters such as introducer catheters with elongate flexible soft distal tip portions to minimise vessel wall trauma. It has been known to provide such distal tip portions as initially separate members that are bonded to the distal end of the catheter tube, with the tip member having filler material therein for viewing by fluoroscopy. The catheter shaft may be of a multiple layer construction using different materials and may include a wire coil to maintain lumen patency. Catheter constructions utilising initially separate distal tip members bonded to a shaft are disclosed in US Patents Nos. 4,898,591; 5,045,072; 5,300,048; 5,548,821 and 5,769,830. However, such tip members are commonly made of copolymers that can be substantially loaded such as by tungsten, barium or bismuth, while the remainder of the catheter shaft contains substantially less radiopaque material adjacent to the distal tip portion.

US 5,045,072 discloses a flexible plastic catheter with a flexible distal tip, which contains sufficient radiopaque agent to be substantially more radiopaque and preferably softer than the portions of the catheter proximal to the tip.

US 5,300,048 describes a flexible plastic material catheter having a proximal portion and a distal end portion having a durometer lower than that of the proximal portion. The plastic material of the distal end portions comprises a homogeneous and evenly dispersed composition of a 20% by weight base thermoplastic elastomer material, such as polyether block amide, and 80% by weight loading of a radiopaque agent, such as tungsten.

It is desired to provide an introducer sheath in which the radiopaque marking is exactly at the distal tip rather than spaced slightly proximally from the tip, to best assure exact positioning by the surgeon.

### Summary of the Invention

According to the invention an introducer sheath comprises a shaft extending from a proximal end portion to a distal end portion; and a distal tip section joined to said shaft at said distal end portion of said shaft; characterised by said shaft and said distal tip section comprising fluorinated ethylene propylene, said distal tip section containing between about 20% and 75% by weight of a radiopaque material selected from the group consisting of tungsten, titanium, tantalum, platinum, gold, silver, bismuth trioxide and lead, and said shaft being distinctly less radiopaque than said distal tip section.

The present invention also is directed to a radiopaque composition of fluorinated ethylene propylene containing a loading of between about 20% to about 75% radiopaque filler, thereby being highly radiopaque, with the filler being tungsten, tantalum, platinum, gold, or lead or other metal.

### Brief Description of the Drawings

An embodiment of the introducer sheath of the present invention will now be described by way of example with reference to the accompanying drawings.
FIGURE 1 is an illustration of a Prior Art introducer sheath containing a metal radiopaque band proximate the distal tip;
FIGURE 2 is an enlarged partial cross-section view of the distal tip region of an introducer sheath containing the present invention; and
FIGURE 3 shows an initially separate tip member with filler.

### Detailed Description

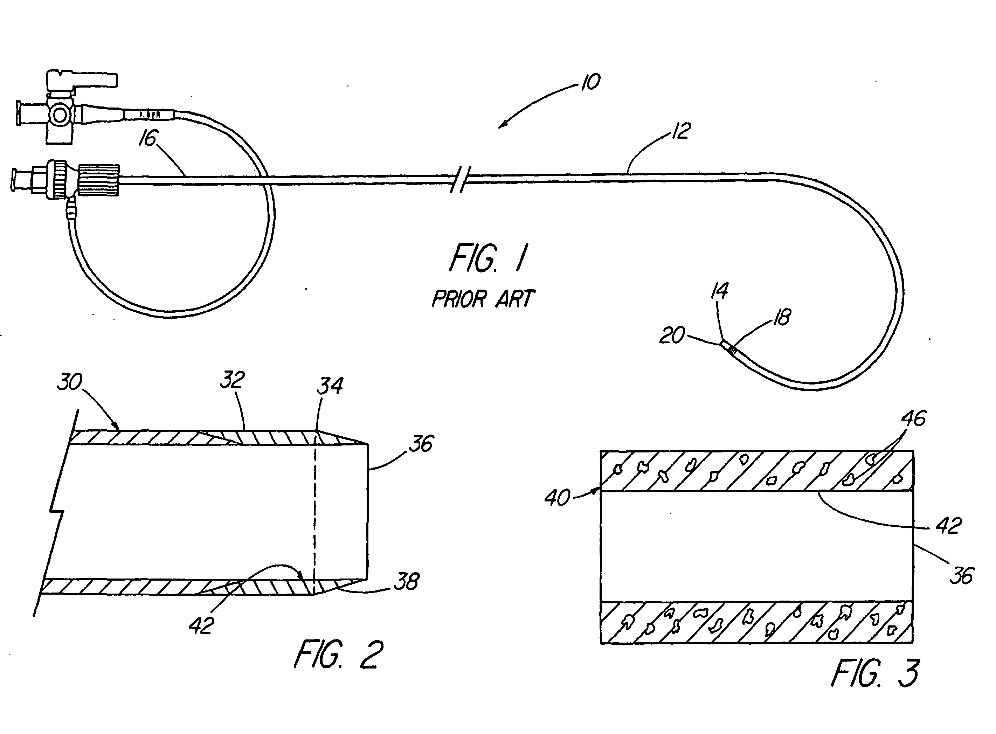

FIG. 1 illustrates an introducer sheath 10 of the prior art, having a shaft 12 having a distal tip 14 and a proximal end 16, and through which extends a lumen. Shaft 12 is polymeric, such as of fluorinated ethylene propylene and contains a radiopaque filler such as an 8 to 12% loading of barium sulphate. Adjacent to the distal end 14 is an annular band 18 of platinum alloy or gold that is highly radiopaque. Distal tip 14 has a tapered outer surface 20 to facilitate insertion into a patient, and metal band 18 embedded within the wall of sheath 10 and is spaced from distal tip 14 about one-quarter inch to assure against becoming dislodged during insertion and removal of the sheath from a patient. During use, a surgeon must estimate the exact location of distal tip 14 distally of the metal band 18, as discerned through fluoroscopy.

FIG. 2 illustrates the distal sheath portion containing the radiopaque distal tip section of the present invention. Sheath shaft 30 includes an end 32, with distal tip section 34 extending distally therefrom to a leading distal end 36 and having a tapered outer surface 38 thereat. Distal tip section 34 may be initially fabricated as a separate member 40 having a lumen 42 equal in diameter of lumen 44 of shaft 30, of a polymeric material that is at least similar enough to the polymeric material of the shaft to be easily and successfully bonded thereto. Such a member is easily extruded and cut to a short length, as shown in FIG. 3.

As an example, member 40 is extruded preferably from fluorinated ethylene propylene having dispersed therein a filler of tungsten particles 46 between about 20% and about 75% by weight, such as preferably about 50 to 55% by weight. The tungsten particles preferably range in size from about 0.5 microns to 25 microns, and more preferably are about 1.4 microns to about 1.8 microns in size. Other polymeric materials include nylon, polyethylene, polyurethane and polytetrafluoroethylene, and other radiopaque filler materials include tantalum, titanium, platinum, gold, silver, bismuth trioxide and lead and the like. It is unexpected that such high loading could be attained with FEP and still result in a stable extrudable composition that can be bonded at least to other FEP material. A loading of 20% tungsten results in a radiopacity that is roughly equivalent to that generated by a 40% loading of barium sulphate.

FEP sheaths have heretofore contained about 5 to 40% barium sulphate filler. Fluorinated ethylene propylene is not known to be fillable to over 40% with barium sulphate particles and still result in a stable extrudable composition. Generally, the particles of barium sulphate used in current introducer sheaths are between about 0.7 microns and 10 microns, preferably about 1 to 3 microns in size. It is believed that an irregular, nonspherical shape of metal particles, along with the high density of the metal, small particle size and narrow size distribution range, may permit such high loading levels in the present invention.

Member 40 can be cut to a length of for example one-quarter inch and be bonded onto an end of shaft 30 such as by adhesive or by thermal bonding, and thereafter be machined for finishing. One such thermal bonding method is disclosed in U.S. Patent No. 5,017,259 for use with catheters. In accordance with U.S. Patent No. 5,769,830, a thermal bond is attained by inserting a mandrel through the tubular shaft and the tip member and then inserted into a forming die to which radiofrequency energy is commonly applied for melting together the materials of the distal end portion of the sheath and the distal tip member.

## Claims

1. An introducer sheath comprising a shaft (30) extending from a proximal end portion to a distal end portion (32); and a distal tip section (34,40) joined to said shaft at said distal end portion (32) of said shaft; **characterised by** said shaft (30) and said distal tip section (34,40) comprising fluorinated ethylene propylene, said distal tip section containing between about 20% and 75% by weight of a radiopaque material selected from the group consisting of tungsten, titanium, tantalum, platinum, gold, silver, bismuth trioxide and lead, and said shaft being distinctly less radiopaque than said distal tip section.

2. The introducer sheath according to claim 1, wherein said distal tip section (34,40) is joined to said shaft (30) by a thermal bond.

3. The introducer sheath according to claim 1, wherein said distal tip section (34, 40) contains between about 50% to 55% by weight of radiopaque material.

4. The introducer sheath according to claim 1, wherein said radiopaque material is tungsten particles (46).

5. The introducer sheath according to claim 4 wherein said tungsten particles (46) range in size from about 0.5 microns to about 25 microns.

6. The introducer sheath according to claim 5, wherein said tungsten particles (46) range in size from about 1.4 microns to about 1.8 microns.

7. The introducer sheath according to claim 6 wherein said distal tip section (34, 40) comprises between about 50% and 55% by weight of said tungsten particles.

8. The introducer sheath according to any of claims 3 to 7, wherein said distal tip section (40) was initially a separate member.

9. The introducer sheath according to any of the preceding claims wherein said distal tip section (34, 40) contains between 50% and 55% by weight of tungsten particles (46) that range in size from about 1.4 microns to about 1.8 microns, said shaft (30) being distinctly less radiopaque than said distal tip section.

## Patentansprüche

1. Einführungshülse, umfassend: Einen Schaft (30), der sich von einem proximalen Endteil zu einem distalen Endteil (32) erstreckt; und einen distalen Spitzenabschnitt (34, 40) verbunden mit dem genannten Schaft am genannten distalen Endteil (32) des genannten Schafts; **gekennzeichnet durch**: Den genannten Schaft (30) und den genannten distalen Spitzenabschnitt (34, 40), umfassend fluoriertes Ethylen-Propylen, den genannten distalen Spitzenabschnitt, enthaltend zwischen etwa 20 und 75 Gewichts-% eines röntgenstrahlenundurchlässigen Materials, ausgewählt aus der Gruppe, bestehend aus: Wolfram, Titan, Tantal, Platin, Gold, Silber, Wismuttrioxid und Blei, und dass der genannte Schaft merklich weniger röntgenstrahlenundurchlässig ist als der genannte distale Spitzenabschnitt.

2. Einführungshülse nach Anspruch 1, worin der genannte distale Spitzenabschnitt (34, 40) mit dem genannten Schaft (30) durch eine thermische Bindung verbunden ist.

3. Einführungshülse nach Anspruch 1, worin der genannte distale Spitzenabschnitt (34, 40) zwischen etwa 50 bis 55 Gewichts-% eines röntgenstrahlenundurchlässigen Materials enthält.

4. Einführungshülse nach Anspruch 1, worin das genannte röntgenstrahlenundurchlässige Material Wolframpartikel (46) ist.

5. Einführungshülse nach Anspruch 4, worin die Größe der genannten Wolframpartikel (46) im Bereich von etwa 0,5 Mikron bis 25 Mikron liegt.

6. Einführungshülse nach Anspruch 5, worin die Größe der genannten Wolframpartikel (46) im Bereich von etwa 1,4 Mikron bis 1 ,8 Mikron liegt.

7. Einführungshülse nach Anspruch 6, worin der genannte distale Spitzenabschnitt (34, 40) zwischen ungefähr 50 und 55 Gewichts-% der genannten Wolframpartikel umfasst.

8. Einführungshülse nach irgendeinem der Ansprüche 3 bis 7, worin der genannte distale Spitzenabschnitt (40) ursprünglich ein separates Teil war.

9. Einführungshülse nach irgendeinem der vorhergehenden Ansprüche, worin der genannte distale Spitzenabschnitt (34, 40) zwischen 50 und 55 Gewichts-% Wolframpartikel (46) enthält, deren Größe im Bereich von etwa 1,4 Mikron bis etwa 1,8 Mikron liegt, und der genannte Schaft (30) merklich weniger röntgenstrahlenundurchlässig ist als der genannte distale Spitzenabschnitt.

## Revendications

1. Gaine d'intubateur comprenant une tige (30) s'étendant entre une partie d'extrémité proximale et une partie d'extrémité distale (32) ; et une section de pointe distale (34, 40) raccordée à ladite tige à ladite partie d'extrémité distale (32) de ladite tige ; **caractérisée en ce que** ladite tige (30) et ladite section de pointe distale (34, 40) comprennent de l'éthylène-propylène fluoré, ladite section de pointe distale contenant entre environ 20% et 75% en poids d'une matière radio-opaque choisie dans le groupe comprenant le tungstène, le titane, le tantale, le platine, l'or, l'argent, le trioxyde de bismuth et le plomb, et ladite tige étant nettement moins radio-opaque que ladite section de pointe distale.

2. Gaine d'intubateur selon la revendication 1, dans laquelle ladite section de pointe distale (34, 40) est raccordée à ladite tige (30) par une liaison thermique.

3. Gaine d'intubateur selon la revendication 1, dans laquelle ladite section de pointe distale (34, 40) contient entre environ 50% et 55% en poids de matière radio-opaque.

4. Gaine d'intubateur selon la revendication 1, dans laquelle ladite matière radio-opaque est constituée par des particules de tungstène (46).

5. Gaine d'intubateur selon la revendication 4, dans laquelle la taille desdites particules de tungstène (46) est comprise entre environ 0,5 micron et environ 25 microns.

6. Gaine d'intubateur selon la revendication 5, dans laquelle la taille desdites particules de tungstène (46) est comprise entre environ 1,4 micron et environ 1,8 micron.

7. Gaine d'intubateur selon la revendication 6, dans laquelle ladite section de pointe distale (34, 40) contient entre environ 50% et 55% en poids desdites particules de tungstène.

8. Gaine d'intubateur selon l'une quelconque des revendications 3 à 7, dans laquelle ladite section de pointe distale (40) est initialement un élément séparé.

9. Gaine d'intubateur selon l'une quelconque des revendications précédentes, dans laquelle ladite section de pointe distale (34, 40) contient entre environ 50% et 55% en poids de particules de tungstène (46) dont la taille est comprise entre environ 1,4 micron et environ 1,8 micron, ladite tige (30) étant nettement moins radio-opaque que ladite section de pointe distale.
